# EUROPÄISCHE PATENTSCHRIFT

(11) **EP 1 994 913 B1**
(45) Veröffentlichungstag und Bekanntmachung des Hinweises auf die Patenterteilung: **15.05.2013**
(21) Anmeldenummer: 08163041.0
(22) Anmeldetag: 28.02.2001
(51) Int. Cl.: A61F 2/24

(54) **Vorrichtung zur Befestigung und Verankerung von Herzklappenprothesen**
Device for fixing and anchoring heart valve prosthetics
Dispositif de fixation et d'ancrage de prothèses de valvule

(30) Priorität: 28.02.2000 DE 10010074
(43) Veröffentlichungstag der Anmeldung: 26.11.2008
(62) Teilanmeldung aus: 01929231.7
(73) Patentinhaber: JenaValve Technology Inc., Wilmington, DE 19801 (US)
(72) Erfinder: Weber, Carsten, 07743, Jena (DE); Peschel, Thomas, 07743, Jena (DE); Damm, Christoph, 07743, Jena (DE); Figulla, Hans-Reiner, 07749, Jena (DE); Ferrari, Markus, 07747, Jena (DE)
(74) Vertreter: Trinks, Ole

(56) Entgegenhaltungen:
- WO-A-91/17720
- US-A- 4 994 077
- US-A- 5 163 953
- US-A- 5 411 552

## Beschreibung

Die Erfindung betrifft eine Vorrichtung zur Befestigung und Verankerung von Herzklappenprothesen, die im Wesentlichen aus drahtförmigen miteinander verbundenen Elementen gebildet ist. Sie kann in zusammengefalteten Zustand minimal invasiv durch die Aorta eingeführt und in der Aortenwand nach Auffaltung verankert werden, so dass die implantierte und befestigte Herzklappenprothese die Funktion der körpereigenen Herzklappe übernehmen kann.

Bisher ist es in nicht ausreichendem Masse gelungen, eine Lösung vorzuschlagen, mit der sowohl eine sichere Abdichtung gegen die Aortenwand, wie auch ein sicherer Halt gewährleistet werden kann. Dabei muss eine solche Vorrichtung bzw. eine solche Verankerungsstütze (Stent) ausreichend klein zusammengefaltet werden können, um dann am Implantationsort aufgespannt zu werden. Bei den bekannten Lösungen wird aber keine ausreichende Vergrößerung mit entsprechender Spannkraft, die den Halt gewährleisten kann, erreicht. Auch Vorschläge bei denen ein Formgedächtnismetall (Memorymetall) verwendet werden soll, genügen nicht den Anforderungen, obwohl mit diesen Materialien bei Erreichen bzw. Überschreiten einer Sprungtemperatur eine Ausdehnung auftritt.

Die Merkmele des Oberbegriffs des Anspruchs 1 sind aus US 5,411,552 bekannt. Auch die in US 5,411,552 beschriebene Lösung kann die Erfordernisse nicht erfüllen, da ein relativ labiles Gebilde verwendet werden soll.

Ein weiteres bisher nur unbefriedigend gelöstes Problem ist die sichere Befestigung einer künstlichen oder biologischen Herzklappenprothese. In der Regel werden die Prothesen aufwendig an einen Stent angenäht. Dies ist zeitaufwendig und muss mit großer Sorgfalt erfolgen, um Beschädigungen zu vermeiden.

Da die implantierten Herzklappenprothesen über große Zeiträume funktionstüchtig sein müssen, spielt auch die konstruktive Ausbildung eine wesentliche Rolle, da es ansonsten nach der Implantation zu Beschädigungen und Undichtheiten kommen kann, die zu lebensbedrohlichen Zuständen des Patienten führen können.

Es ist daher Aufgabe der Erfindung eine Vorrichtung zur Befestigung und Verankerung von Herzklappenprothesen vorzuschlagen, die für eine minimal-invasive Implantion durch die Aorta klein zusammengefaltet und am Implantationsort entfaltet werden kann, wobei ein sicherer Halt und eine sichere Abdichtung gegenüber der Aortenwand gewährleistet ist.

Erfindungsgemäß wird diese Aufgabe mit einer Vorrichtung nach Anspruch 1 gelöst.

Vorteilhafte Ausgestaltungsformen und Weiterbildungen der Erfindung können mit den in den untergeordneten Ansprüchen genannten Merkmalen erreicht werden.

Wesentliche Elemente der erfindungsgemäßen Lösung sind jeweils drei gleiche Paare von Bügeln, die in jeweils um 120 ° versetzter Anordnung miteinander verbunden sind. Die beiden Bügel eines Paares sind entgegengesetzt zueinander gekrümmt gebogen und mittels Festkörpergelenken verbunden. Die Festkörpergelenke erfüllen gleichzeitig die Funktion von Drehlagern, um die die Bügel eines Paares, ähnlich wie bei einer Wippe, verschwenkt werden können. Wird auf einen der Bügel eine Druckkraft, z. B. durch Peristaltik der Aorta ausgeübt, wird dieser Bügel entsprechend in die gleiche Richtung um die Drehachse am Festkörpergelenk verschwenkt. Gleichzeitig wird der jeweils andere Bügel des Paares entgegengesetzt dazu verschwenkt. Demzufolge wird dann immer einer der beiden Bügel gegen die Aortenwand gedrückt, was die Abdichtung und den Halt erhöht.

Günstig ist es, die Bügel eines Paares so zu dimensionieren, dass möglichst gleiche Hebelverhältnisse in Bezug zu den die Drehlager bildenden Festkörpergelenken eingehalten werden, also gleich lange zumindest annähernd gleich lange Hebelarme gebildet werden.

Vorteilhaft sind auch die durch die 120 ° Anordnung der Bügelpaare vorgegebenen relativ großen Abstände der Festkörpergelenke und die ebenfalls von den Bügeln abgedeckten großen Flächenbereiche.

Wobei die distalen Bügel nicht nur zur Befestigung der Herzklappenprothese dienen, sondern auch Stützfunktion übernehmen.

Durch einen weiteren in distaler Richtung angeordneten und gekrümmten Bügel können die genannten Vorteile noch verbessert werden.

Dabei ist der zweite distale Bügel in seinem distalen Bereich annähernd, wie der erste distale Bügel gekrümmt ausgebildet. Teilweise sind diese beiden Bügel so ausgebildet und geformt, dass sie nebeneinander verlaufen und zwischen ihnen Spalte ausgebildet sind. Sie können an der gleichen Stelle, an dem auch die Festkörpergelenke, als Verbindung zum in proximaler Richtung gekrümmten Bügel angeordnet sind, miteinander verbunden sein. Die gebildeten Spalte sind daher in distaler Richtung offen und es können Teile der Herzklappenprothese in die Spalte eingeführt und gehalten werden.

Zumindest ein Teil eines distalen Bügels ist proximal eingezogen und bis zu einem Wendepunkt geführt in dem benachbarte Bügel zusammengeführt sind. Bei zwei distal angeordneten Bügeln trifft dies auf den jeweils distal äußeren Bügel zu.

Zur Versteifung und für eine weitere Befestigungsmöglichkeit der Herzklappenprothese kann ein winkelförmig gekrümmter, ebenfalls proximal eingezogener Bügel verwendet werden, dessen gekrümmte Teile zwischen jeweils benachbarten Bügeln angeordnet und der jeweiligen Krümmung teilweise folgend ausgebildet sind. Diese Bügel sind mit ihren jeweils distalen Enden an dem einen distal äußeren oder dem jeweils distal äußeren Bügel befestigt. Auch hier bildet die Befestigung jeweils ein Festkörpergelenk. Diese sollten jedoch in einem Abstand zu den anderen, die Bügel eines Paares verbindenden Festkörpergelenken angeordnet sein.

Bei implantierter, aufgespannter Vorrichtung können dann die Taschen einer Herzklappenprothese dort eingeschoben, gehalten und gestützt werden.

Die Bügelkonstruktion der erfindungsgemäßen Vorrichtung stützt eine Herzklappenprothese großflächig und demzufolge schonend ab. Außerdem kann sie mit wesentlich geringerem Aufwand, beispielsweise durch Nähen befestigt werden.

Die konstruktive Lösung ermöglicht einen sicheren Halt und die erforderliche Abdichtung an bzw. gegenüber der Aortenwand. Für die Abdichtung und eine verringerte Belastung für die Herzklappenprothese wirkt sich auch das Anpressen der Herzklappenprothese von innen mittels der Bügel günstig aus.

Die erfindungsgemäße Vorrichtung kann mit einem Ballonkatheter implantiert und am Implantationsort entfaltet werden. Vorteilhaft wird für die Vorrichtung ebenfalls ein Formgedächtnismetall mit geeigneter Sprungtemperatur verwendet, mit dem zusätzlich eine Dehnung erreicht werden kann. Hierfür kann eine Nickel- und Titan enthaltende Legierung, die unter der Bezeichnung Nitinol verfügbar ist, verwendet werden.

Außerdem kann der die Herzklappenprothese tragende und haltende Teil der Vorrichtung separat zu einem Stützkörper, auf den nachfolgend, bei der Beschreibung eines Ausführungsbeispieles noch Bezug genommen wird, implantiert werden, ohne dass die vorteilhaften Eigenschaften vermindert werden. Die Implantation dieses im Wesentlichen aus den drei Segmenten mit daran befestigter Herzklappenprothese bestehenden Teiles, kann dann in herkömmlicher Form operativ erfolgen.

Nachfolgend soll die Erfindung an einem Ausführungsbeispiel näher erläutert werden.

Dabei zeigt:

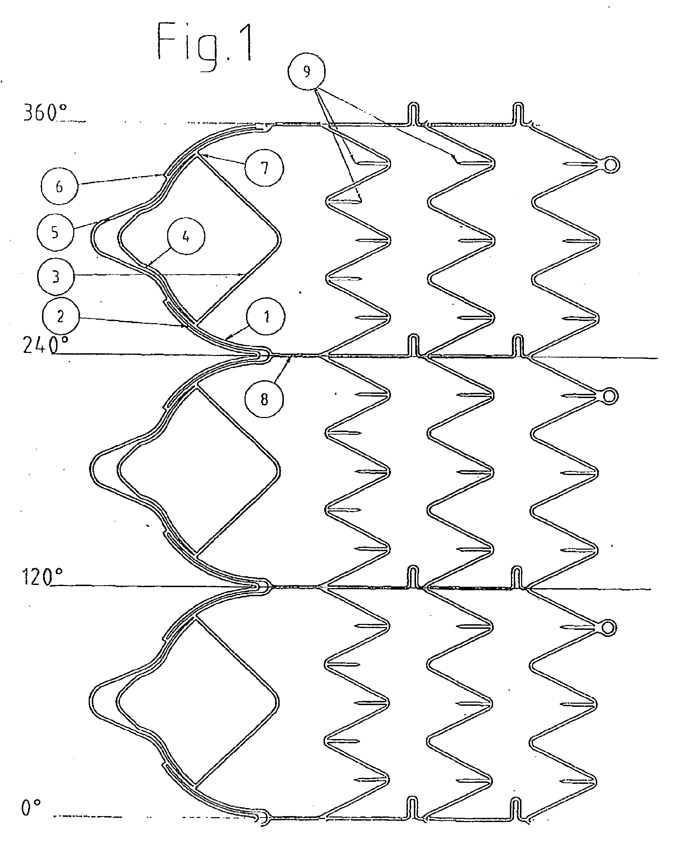

Figur 1 eine Abwicklung eines Beispiels einer erfindungsgemäßen Vorrichtung.

In der Figur 1 ist eine Abwicklung eines Beispiels einer erfindungsgemäßen Vorrichtung gezeigt. Die Vorrichtung ist radial symmetrisch ausgebildet, wobei drei gleiche Teile in einer 120 ° Anordnung verwendet werden.

Jedes Teil verwendet eine Bügelkonstruktion als Träger und zur Befestigung einer künstlichen oder biologischen Herzklappenprothese.

Bei diesem Beispiel werden zwei Bügel 4 und 5 verwendet, die distal außen angeordnet sind, wobei ggf. auf den äußeren Bügel 5 verzichtet werden könnte.

Der gekrümmt gebogene Bügel 4 ist mit einem in entgegengesetzter Richtung gebogenen Bügel 3 verbunden. Die beidseitigen Verbindungen bilden Festkörpergelenke 7, die gleichzeitig Drehlagerfunktion für die beiden Hebel darstellenden Bügel 3 und 4 übernehmen, wie bereits im allgemeinen Teil der Beschreibung erklärt.

Der zweite nach außen gebogene und distal angeordnete Bügel 3 erhöht die Stabilität und bietet eine zusätzliche Abstütz- und Befestigungsmöglichkeit für die Herzklappenprothese. Dabei sind die beiden distal äußeren Bügel 4 und 5 ebenfalls miteinander verbunden, wobei die Verbindung an gleicher Stelle, an dem auch die Festkörpergelenke 7 angeordnet sind, erfolgen kann.

Zwischen den beiden Bügeln 4 und 5 sind aus distaler Richtung offene Spalten vorhanden, in die Teile der Herzklappenprothese eingeführt und dort fixiert werden können.

Der hier äußere Bügel 5 ist in proximaler Richtung weiter nach innen gezogen und mit seinem Ende mit jeweils einem Stützsteg 8 verbunden. Die Stützstege 8 sind bei diesem Beispiel parallel zur Längsachse der Vorrichtung ausgerichtet und sie bilden gemeinsam mit sägezahn-, rauten- oder mäanderförmigen Querstegen einen Stützkörper, der im entfalteten Zustand an der Aortenwand anliegt. Zur Verhakung können zusätzliche Spitzen 9 an den Stützstegen 8 und/oder den Querstegen vorhanden bzw. ausgebildet sein, die sich in der Aortenwand verhaken.

Die Anordnung und Länge der Stützstege 8 und der entsprechend große Abstand zur im Bereich der Bügel 3,4 und 5 befestigten Herzklappenprothese, ermöglichen das Positionieren der Herzklappenprothese ohne die Herzkranzgefäße zu verschließen bzw. abzudecken.

Bei dem hier gezeigten Beispiel sind zwischen den einzelnen in 120 ° Anordnung verwendeten Segmenten zusätzliche proximal eingezogene Bügel 2 vorhanden, die mit den distal äußeren Bügeln 5 verbunden sind. Auch hierbei sind die Verbindungen Festkörpergelenke 6, die jedoch möglichst in einem Abstand zu den Festkörpergelenken 7 angeordnet sein sollten. Es können so zwei Hebel pro Segment genutzt und mit einem solchen doppelt gespiegelten Aufbau in etwa doppelt große Kräfte realisiert werden, um die Vorrichtung zu fixieren.

Im entfalteten implantierten Zustand können zwischen den Teilen 1 der Bügel 5 und den Bügeln 2 wiederum Teile der Herzklappenprothese wechselweise eingeführt, so gestützt und daran befestigt werden.

Die Anzahl der verwendeten Bügel kann aber noch erhöht werden, um den Halt zu verbessern und die Belastung der Herzklappenprothese weiter zu verringern.

## Patentansprüche

1. Vorrichtung zur Befestigung und Verankerung von Herzklappenprothesen, die aus drahtförmigen Elementen gebildet ist, wobei die Vorrichtung einen Stent bestehend aus drei gleichen Bügelkonstruktionen (3, 4, 5) in einer 120° Anordnung aufweist, **dadurch gekennzeichnet, daß** jede Bügelkonstruktion (3, 4, 5) folgendes aufweist:
- einen ersten nach außen gebogenen und an einem ersten Ende des Stents angeordneten Bügel (4) sowie einen zweiten ebenfalls nach außen gebogenen und an dem ersten Ende des Stents angeordneten Bügel (5); und
- einen in Bezug auf den ersten und zweiten Bügel (4, 5) in entgegengesetzter Richtung gebogenen Bügel (3), der mit dem ersten nach außen gebogenen Bügel (4) verbunden ist,
wobei der Stent ferner Stützstege (8) aufweist, die mit den Enden der weiter zum zweiten Ende des Stents hin gezogenen zweiten Bügel (5) verbunden sind und parallel zur Längsachse in Richtung des zweiten Ende des Stents verlaufen, wobei die Stützstege (8) miteinander über sägezahn-, rauten-, oder mäanderförmig ausgebildete Querstege verbunden sind, zur Ausbildung eines Stützkörpers am zweiten Ende des Stents.

2. Vorrichtung nach Anspruch 1,
wobei die ersten und zweiten nach außen gebogenen Bügel (4, 5) einer jeden Bügelkonstruktion (3, 4, 5) relativ zueinander so angeordnet sind, dass zwischen ihnen ein Spalt vorliegt, in den Teile einer an dem Stent zu befestigenden Herzklappenprothese einführbar und dort fixierbar sind.

3. Vorrichtung nach einem der vorherigen Ansprüche,
wobei der Stent von einem zusammengefalteten Zustand während der Implantation in einen entfalteten Zustand im Implantationsort überführbar ist.

4. Vorrichtung nach Anspruch 3,
wobei der Stent selbst-expandierbar ausgebildet ist und aus einem Formgedächtnismaterial mit geeigneter Sprungtemperatur besteht.

5. Vorrichtung nach einem der vorhergehenden Ansprüche,
wobei ferner zusätzliche Spitzen (9) an den Stützstegen (8) und/oder den Querstegen ausgebildet sind zur Verankerung des Stents im Implantationsort.

6. Vorrichtung nach einem der vorherigen Ansprüche,
wobei am zweiten Ende des Stents insgesamt drei Ösen ausgebildet sind, die gleichmäßig über den Stentumfang verteilt sind.

7. Vorrichtung nach einem der vorherigen Ansprüche,
wobei ferner eine Herzklappenprothese vorgesehen ist, welche an den jeweiligen Bügelkonstruktionen (3, 4, 5) durch Nähen befestigt ist.

8. Vorrichtung nach Anspruch 7,
wobei die Herzklappenprothese eine künstliche oder eine biologische Herzklappenprothese ist.

## Claims

1. A device for fastening and anchoring heart valve prostheses formed of wire-shaped elements, wherein the device comprises a stent consisting of three equal bow-shaped structures (3, 4, 5) in a 120° arrangement, **characterized in that** each bow structure (3, 4, 5) comprises the following:
- a first bow element (4) bent outward and disposed on a first end of the stent as well as a second bow element (5) likewise bent outward and disposed on the first end of the stent; and
- a bow element (3) bent in the opposite direction as that of the first and second bow element (4, 5) and connected to the first outwardly bent bow element (4),
wherein the stent further comprises support bars (8) connected to the ends of the second bow element (5) pulled further toward the second end of the stent and which run parallel to the longitudinal axis toward the second end of the stent, wherein the support bars (8) are connected together by means of sawtooth-shaped, diamond-shaped or zigzag crossbars to form a support body on the second end of the stent.

2. The device according to claim 1,
wherein the first and second outwardly bent bow elements (4, 5) of a respective bow structure (3, 4, 5) are arranged relative each other such that a gap is formed between them in which parts of a heart valve prosthesis to affix to a stent can be introduced and secured there.

3. The device according to any one of the preceding claims,
wherein during implantation, the stent can be transformed at the implantation site from a collapsed state into an unfolded state.

4. The device according to claim 3,
wherein the stent is of self-expanding design and consists of a shape memory material having the applicable transition temperature.

5. The device according to any one of the preceding claims,
wherein nibs (9) are further configured on the support bars (8) and/or the crossbars to anchor the stent at the implantation site.

6. The device according to any one of the preceding claims,
wherein a total of three eyelets are configured on the second end of the stent which are evenly distributed over the circumference of the stent.

7. The device according to any one of the preceding claims,
wherein a heart valve prosthesis is further provided which is affixed to the respective bow structures (3, 4, 5) by suturing.

8. The device according to claim 7,
wherein the heart valve prosthesis is an artificial or biological prosthetic heart valve.

## Revendications

1. Dispositif pour fixer et ancrer des prothèses de valvules cardiaques, qui est formé par des éléments de forme filaire, dans lequel le dispositif comprend un stent constitué de trois constructions en arceau identiques (3, 4, 5) dans un agencement à 120°,
**caractérisé en ce que** chaque construction en arceau (3, 4, 5) comprend les éléments suivants :
- un premier arceau (4) cintré vers l'extérieur et agencé à une première extrémité du stent ainsi qu'un deuxième arceau (5) également cintré vers l'extérieur et agencé à la première extrémité du stent; et
- un arceau (3) cintré en direction opposée au premier et au second arceau (4, 5), qui est relié au premier arceau (4) cintré vers l'extérieur,
dans lequel le stent comprend en outre des barrettes de soutien (8), qui sont reliées avec les extrémités du second arceau (5), tirées plus loin vers la seconde extrémité du stent, et qui s'étendent parallèlement à l'axe longitudinal en direction de la seconde extrémité du stent, et les barrettes de soutien (8) sont reliées les unes aux autres via des barrettes transversales réalisées en forme de dents de scie, en forme de losange ou en forme de méandres, pour réaliser un corps de soutien à la seconde extrémité du stent.

2. Dispositif selon la revendication 1,
dans lequel le premier et le second arceau (4, 5) cintrés vers l'extérieur de chaque construction en arceau (3, 4, 5) sont agencés les uns par rapport aux autres de telle façon qu'il existe un intervalle entre eux, dans lequel des parties d'une prothèse de valvule cardiaque à fixer au stent peuvent être introduites et être fixées à cet endroit.

3. Dispositif selon l'une des revendications précédentes,
dans lequel le stent peut être transféré depuis une situation repliée sur lui-même pendant l'implantation jusque dans une situation déployée dans le lieu d'implantation.

4. Dispositif selon la revendication 3,
dans lequel le stent est réalisé autoexpansible et est constitué d'un matériau à mémoire de forme avec une température de déclenchement appropriée.

5. Dispositif selon l'une des revendications précédentes,
dans lequel des pointes supplémentaires (9) sont en outre réalisées sur les barrettes de soutien (8) et/ou les barrettes transversales pour l'ancrage du stent dans le lieu d'implantation.

6. Dispositif selon l'une des revendications précédentes,
dans lequel au total trois oeillets sont réalisés à la seconde extrémité du stent, qui sont régulièrement répartis sur la périphérie du stent.

7. Dispositif selon l'une des revendications précédentes,
dans lequel il est prévu une prothèse de valvule cardiaque qui est fixée aux constructions en arceau respectives (3, 4, 5) par couture.

8. Dispositif selon la revendication 7,
dans lequel la prothèse de valvule cardiaque est une prothèse de valvule cardiaque artificielle ou biologique.
